# EUROPÄISCHE PATENTSCHRIFT

(11) **EP 0 955 342 B1**
(45) Veröffentlichungstag und Bekanntmachung des Hinweises auf die Patenterteilung: **11.07.2001**
(21) Anmeldenummer: 99107083.0
(22) Anmeldetag: 12.04.1999
(51) Int. Cl.: C09B 44/02, C09B 35/21, C09B 35/378, C07C 229/56, C07C 237/34, C09B 67/22

(54) **Kationische Azofarbstoffe auf der Basis von Aminobenzoesäure**
Cationic azo dyes derived from aminobenzoic acid
Colorants azoiques cationiques dérivés de l'acide aminobenzoique

(30) Priorität: 07.05.1998 DE 19820400
(43) Veröffentlichungstag der Anmeldung: 10.11.1999
(73) Patentinhaber: BASF AKTIENGESELLSCHAFT, 67056 Ludwigshafen (DE)
(72) Erfinder: Etzbach, Karl-Heinz Dr., 67227 Frankenthal (DE); Freund, Torsten Dr., 67117 Limburgerhof (DE); Tresch, Rainer, 67133 Maxdorf (DE)

(56) Entgegenhaltungen:
- EP-A- 0 159 549
- EP-A- 0 162 409
- DE-A- 3 821 627

## Beschreibung

Die vorliegende Erfindung betrifft neue kationische Azofarbstoffe der allgemeinen Formel I 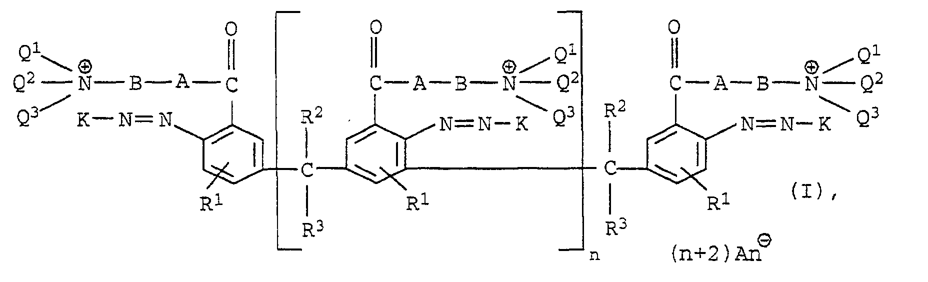 in der
- n: eine Zahl von 0 bis 8,
- R¹: Wasserstoff, Nitro, Halogen, C₁-C₆-Alkyl,
- R² und R³: unabhängig voneinander Wasserstoff, C₁-C₆-Alkyl oder zusammen mit dem sie verbindenden Kohlenstoffatom einen Cyclopentyl- oder Cyclohexylrest,
- K: den Rest einer Kupplungskomponente,
- A: Oxy, Imino oder gegebenenfalls substituiertes C₁-C₆-Alkylimino,
- B: C₁-C₆-Alkylen, das durch 1 oder 2 nichtbenachbarte Oxy-, Imino-, gegebenenfalls substituierte C₁-C₆-Alkyliminogruppen und einem Rest 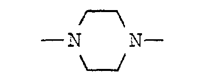
unterbrochen sein kann,
- Q¹, Q² und Q³: unabhängig voneinander Wasserstoff, C₁-C₁₀-Alkyl, das durch 1 bis 4 nichtbenachbarte Oxy-, Imino- und gegebenenfalls substituierte C₁-C₄-Alkyliminogruppen unterbrochen sein kann und das mit Hydroxy, Halogen oder Phenyl substituiert sein kann und Q¹ und Q² zusammen mit dem sie verbindenden Stickstoffatom einen 5- oder 6-gliedrigen heterocyclischen Rest, der gegebenenfalls Stickstoff oder Sauerstoff als weiteres Heteroatom aufweist, und
- An^{⊖}: das Äquivalent eines Anions bedeuten,

Amine als ihre Zwischenprodukte, ein Verfahren zu ihrer Herstellung, ihre Verwendung zum Färben und Bedrucken von natürlichen oder synthetischen Substraten sowie ihre Mischungen.

Die EP-A-0 162 409 beschreibt basische Azofarbstoffe, die durch einfache oder doppelte Kupplung von Anthranilsäurealkylester, deren Alkylrest alkylaminosubstituiert ist, auf Resorcin erhalten wurden. Diese Farbstoffe weisen jedoch in ihrer Verwendung als Papierfarbstoffe Mängel in Affinität und Farbstärke auf.

Daher war es Aufgabe der vorliegenden Erfindung, neue Farbstoffe bereitzustellen, die sich durch vorteilhafte anwendungstechnische Eigenschaften, insbesondere gute Lichtechtheiten, sowie hohe Farbstärke und Affinität zur Faser auszeichnen. Außerdem sollen die gefärbten Fasern gute Echtheiten gegenüber Bleichmitteln zeigen.

Demgemäß wurden die eingangs näher bezeichneten Farbstoffe der Formel I gefunden.

Alle in der obengenannten Formel auftretenden Alkyl- oder Alkylengruppen können sowohl geradkettig als auch verzweigt sein.

Wenn in der obengenannten Formel substituierte Alkylgruppen auftreten, so kommen als Substituenten z.B. Hydroxy und Methoxy in Betracht. Die Alkylgruppen weisen dann in der Regel 1 bis 2 Substituenten auf.

Wenn die Reste Q¹ und Q² zusammen mit dem sie verbindenden Stickstoffatom für einen 5- oder 6-gliedrigen heterocyclischen Rest stehen, der gegebenenfalls Stickstoff oder Sauerstoff als weiteres Heteroatom aufweist, so können gesättigte Reste wie Pyrrolidinyl, Piperidinyl, Morpholinyl, Piperazinyl, das am Stickstoff mit Methyl, Ethyl, Propyl, Isopropyl, n-, iso- und sec.-Butyl, 2-Hydroxyethyl oder 2- oder 3-Hydroxypropyl substituiert sein kann, in Betracht kommen. Ferner können ungesättigte Reste wie Pyrrolyl, Pyrazolyl, Oxazolyl, Isooxazolyl oder Imidazolyl, das in 2- und/oder 4-Stellung durch Methyl, Ethyl, Propyl oder Butyl substituiert sein kann oder N-3-(C₁-C₄)-Alkylimidazolyl, das in 2- und/oder 4-Stellung noch mit Methyl, Ethyl, Propyl oder Butyl substituiert sein kann, in Betracht kommen.

Reste Halogen sind Fluor, Chlor oder Brom.

Reste R¹, R², R³, Q¹, Q² und Q³ sind z.B. Methyl, Ethyl, Propyl, Isopropyl, Butyl, Isobutyl, sec.-Butyl, tert.-Butyl, Pentyl, Isopentyl, Neopentyl, tert.-Pentyl, Hexyl oder 2-Methylpentyl.

Reste Q¹, Q² und Q³ sind weiterhin z.B. Heptyl, Octyl, 2-Ethylhexyl, Isooctyl, Nonyl, Isononyl, Decyl (die obigen Bezeichnungen Isooctyl, Isononyl und Isodecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), 2-Hydroxyethyl, 2- oder 3-Hydroxypropyl, 2- oder 4-Hydroxybutyl, 2-Chlorethyl, 2- oder 3-Chlorpropyl, 2- oder 4-Chlorbutyl, Benzyl, 1- oder 2-Phenylethyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2-oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl, 3,6,9,12-Tetraoxatetradecyl, 4-Hydroxy-2-methyl-3-azabutyl, 4-Hydroxy-3-hydroxymethyl-2-methyl-3-azabutyl, 5-Hydroxy-2-methyl-3-azapentyl, 5-Hydroxy-3-(2-hydroxyethyl)-2-methyl-3-azapentyl, 8-Hydroxy-2-methyl-3-aza-6-oxaoctyl, 11-Hydroxy-2-methyl-3-aza-6,9-dioxaundecyl, 8-Hydroxy-(5-hydroxy-3-oxapentyl)-2-methyl-3-aza-6-oxaoctyl oder 11-Hydroxy-3-(8-hydroxy-3,6-dioxaoctyl)-2-methyl-3-aza-6,9-dioxaundecyl, N,N-Dimethylaminoethyl, N,N-Diethylaminoethyl, N,N-Di-propylaminoethyl, N,N-Dibutylaminoethyl, 3-(N,N-Dimethylamino)-propyl, 3-(N,N-Diethylamino)-propyl, 3-(N,N-Dipropylamino)-propyl oder 3-(N,N-Dibutylamino)-propyl.

Reste A sind z.B. Methylimino, Ethylimino, Propylimino, Isopropylimino, Butylimino, Isobutylimino, sec.-Butylimino, tert.-Butylimino, Pentylimino, Isopentylimino, Neopentylimino, tert.-Pentylimino, Hexylimino oder 2-Methylpentylimino.

Reste B sind z.B. Methylen, Ethylen, 1,2- oder 1,3-Propylen, 1,2-, 2,3- oder 1,4-Butylen, (CH₂)₂O(CH₂)₂, (CH₂)₃O(CH₂)₂, (CH₂)₃O(CH₂)₃, (CH₂)₂O(CH₂)₂O(CH₂)₂, (CH₂)₂NH(CH₂)₂, (CH₂)₃NH(CH₂)₂, (CH₂)₂NH(CH₂)₂NH(CH₂)₂,

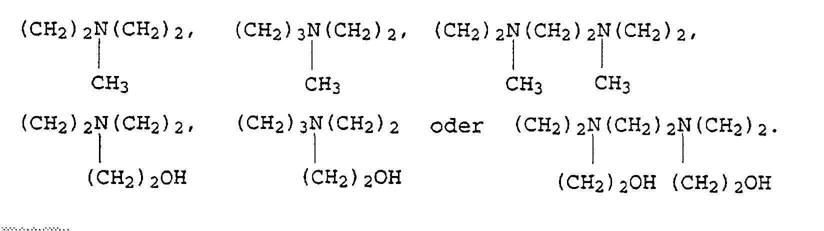

Das Äquivalent An⊖ eines Anions leitet sich z.B. von folgenden Anionen ab: Fluorid, Chlorid, Bromid, Iodid, Sulfat, Phosphat, Formiat, Acetat, Propionat, Mono-, Di- oder Trichloracetat, Lactat, Methoxyacetat, Citrat, Succinat, Methansulfonat, Benzolsulfonat, 2- oder 4-Methylbenzolsulfonat oder Naphthalinsulfonat.

Als Kupplungskomponenten KH sind Verbindungen aus der Benzol-, Naphthalin-, Chinolin-, Pyridon-, Barbitursäure- oder Pyrazolonreihe geeignet und entsprechen beispielsweise den Verbindungen der Formeln II a-f 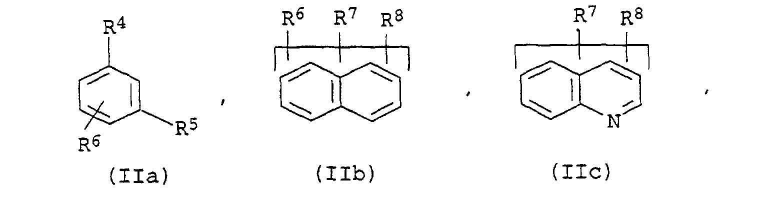 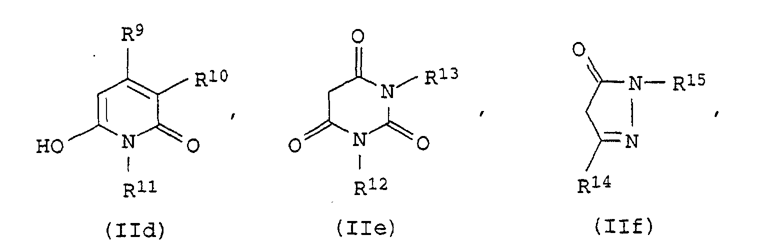 worin
- R⁴: Hydroxy, Amino, Morpholino, Mono- oder Di-(C₁-C₆)-alkylamino, deren Alkylreste gegebenenfalls mit Hydroxy, Amino, Cyano, C₁-C₆-Alkoxycarbonyl, Carbamoyl oder Mono- oder Di-(C₁-C₆)-alkylcarbamoyl substituiert ist und gegebenenfalls durch Oxy unterbrochen ist, oder C₁-C₆-Alkoxy,
- R⁵: Wasserstoff, C₁-C₆-Alkyl oder einen Rest R⁴,
- R⁶: Wasserstoff, C₁-C₆-Alkyl, C₁-C₆-Alkoxy, dessen Alkylrest gegebenenfalls durch Oxy unterbrochen ist, Mono- oder Di-(C₁-C₆)-alkyimino, C₁-C₆-Alkoxycarbonyl, Carbamoyl, Mono- oder Di-(C₁-C₆)-alkylcarbamoyl, deren Alkylreste gegebenenfalls mit Hydroxy oder Amino substituiert sind und gegebenenfalls durch Oxy unterbrochen sind, Sulfamoyl, Mono- oder Di-(C₁-C₆)-alkylsulfamoyl, deren Alkylreste gegebenenfalls mit Hydroxy oder Amino substituiert sind und gegebenenfalls durch Oxy unterbrochen sind, oder Carboxyl,
- R⁷: Hydroxy, Amino, Mono- oder Di-(C₁-C₁₂)-alkylamino, Cyclohexylamino,
- R⁸: Wasserstoff, Hydroxy, Amino, Formylamino, Acetylamino, C₁-C₆-Alkyl,
- R⁹: Wasserstoff, C₁-C₆-Alkyl, Phenyl, Hydroxyl, Cyano, Acetyl, Benzoyl, Methoxycarbonyl, Carbamoyl,
- R¹⁰: Wasserstoff, Chlor, Brom, Acetylamino, Amino, Nitro, Sulfamoyl, Methylsulfonyl, Phenylsulfonyl, C₁-C₄-Alkoxycarbonyl, C₁-C₄-Alkanoyl, Benzoyl, Carbamoyl, Cyano, N-Methylimidazolyl, Pyridinio,
- R¹¹: Wasserstoff, C₁-C₆-Alkyl, das durch Phenyl, Hydroxy, Amino, C₁-C₆-Alkoxyl, Acetylamino, Benzoylamino oder Cyano substituiert sein kann, Cyclohexyl, Phenyl, das durch Benzoylamino, Acetylamino, Methyl, Methoxy, Cyano oder Chlor substituiert sein kann, oder Amino, das durch Phenyl, C₁-C₆-Alkyl, C₁-C₆-Alkanoyl oder Benzoyl substituiert ist,
- R¹² oder R¹³: unabhängig voneinander Wasserstoff oder C₁-C₆-Alkyl bedeuten,
- R¹⁴: Methyl, C₁-C₄-Alkoxycarbonyl oder Phenyl,
- R¹⁵: Wasserstoff, C₁-C₆-Alkyl, Cyclohexyl, Benzyl oder Phenyl, das gegebenenfalls ein- bis dreifach mit Fluor, Chlor, Brom, C₁-C₄-Alkyl, C₁-C₄-Alkoxy, Cyano, Acetyl, Acetylamino, Hydroxy, Sulfamoyl oder Carbamoyl substituiert ist, bedeuten.

Reste R⁴, R⁶ und R⁷ sind z.B. Mono- oder Dimethylamino, Mono- oder Diethylamino, Mono- oder Dipropylamino, Mono- oder Diisopropylamino, Mono- oder Dibutylamino, Mono- oder Dipentylamino, Monooder Diisopentylamino, Mono- oder Dineopentylamino, Mono- oder Dihexylamino, Mono- oder Di-2-methylpentylamino, N-Methyl-n-butylamino, N-Ethyl-n-butylamino.

Reste R⁴ sind weiterhin z.B. Mono- oder Di-(2-hydroxyethyl)amino, Mono- oder Di-(2-hydroxypropyl)amino, Mono- oder Di-(3-hydroxypropyl)amino, N-Methyl-2-hydroxyethylamino, N-Methyl-2-hydroxypropylamino, N-Methyl-3-hydroxypropylamino, N-Ethyl-2-hydroxyethylamino, N-Ethyl-2-hydroxypropylamino, N-Ethyl-3-hydroxypropylamino, N-Propyl-2-hydroxyethylamino, N-Propyl-2-hydroxypropylamino, N-Propyl-3-hydroxypropylamino, N-Isopropyl-2-hydroxyethylamino, -2-hydroxypropylamino, -3-hydroxypropylamino, Mono- oder Di-(2-aminoethyl)amino, Mono- oder Di-(2-aminopropyl)amino, Mono- oder Di-(3-aminopropyl)amino, Mono- oder Di-(2-aminobutyl)amino, Mono- oder Di-(4-aminobutyl)amino, 2-Cyanoethylamino, 2-Cyano-2-methylethylamino, 2-Methoxycarbonylethylamino, 2-Methoxycarbonyl-2-methylethylamino, 2-Ethoxycarbonylethylamino, 2-Ethoxycarbonyl-2-methylethylamino, 2-Propoxycarbonylethylamino, 2-Propoxycarbonyl-2-methylethylamino, 2-Isopropoxycarbonylethylamino, 2-Isopropoxycarbonyl-2-methylethylamino, 2-Butoxycarbonylethylamino, 2-Butoxycarbonyl-2-methylethylamino, 2-Carbamoylethylamino, 2-Carbamoyl-2-methylethylamino, 2-Mono- oder Dimethylaminocarbonylethylamino, 2-Mono- oder Dimethylaminocarbonyl-2-methylethylamino, Monooder Di-(2-methoxyethyl)amino, Mono- oder Di-(2-methoxypropyl)amino, Mono- oder Di-(3-methoxypropyl)amino, oder Monooder Di-(2-hydroxyethyloxyethyl)amino.

Reste R⁵, R⁶, R⁸, R⁹, R¹¹, R¹², R¹³ und R¹⁵ sind z.B. Methyl, Ethyl, Propyl, Isopropyl, Butyl, Isobutyl, sec-Butyl, tert-Butyl, Pentyl, Isopentyl, Neopentyl, tert.-Pentyl, Hexyl oder 2-Methylpentyl.

Die Reste R¹¹ sind Hydroxy-C₁-C₄-alkyl wie Hydroxymethyl, 1-Hydroxyeth-1-yl, 2-Hydroxyeth-1-yl, 1-Hydroxy-prop-1-yl, 2-Hydroxyprop-1-yl, 3-Hydroxyprop-1-yl, 1-Hydroxy-prop-2-yl, 2-Hydroxyprop-2-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 1-Hydroxybut-3-yl, 2-Hydroxybut-3-yl, 1-Hydroxy-2-methyl-prop-3-yl, 2-Hydroxy-2-methyl-prop-3-yl, 3-Hydroxy-2-methyl-prop-3-yl, 2-Hydroxymethyl-prop-2-yl, Cyanomethyl, Cyanoethyl, Cyanopropyl oder Cyanobutyl.

Reste R¹¹ sind weiterhin z.B. Benzyl, 1-Phenylethyl, 2-Phenylethyl, 1-Phenylprop-1-yl, 2-Phenylprop-1-yl, 3-Phenylprop-1-yl. 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl, 1-(Phenylmethyl)-prop-l-yl, vorzugsweise Benzyl und 2-Phenylethyl, Methoxymethyl, Ethoxymethyl, n-Propoxy-methyl, (1-Methylethoxy)methyl, n-Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(n-Propoxy)ethyl, 2-(1-Methoxyethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)-propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl.

Reste R¹⁰ sind z.B. Formyl, Acetyl, Propionyl, Butyryl oder Isobutyryl.

Die Reste R⁶ sind beispielsweise Mono- oder Dimethylcarbamoyl, Mono- oder Diethylcarbamoyl, Mono- oder Dipropylcarbamoyl, Mono-oder Dibutylcarbamoyl, Mono- oder Dipentylcarbamoyl oder Mono-oder Dihexylcarbamoyl.

Reste R⁴ und R⁶ können z.B. Methoxy, Ethoxy, Propoxy, Isopropoxy, Butoxy, sec-Butoxy, Isobutoxy oder tert-Butoxy sein.

Reste R⁶ sind weiterhin z.B. Methoxyethyloxy, Methoxypropyloxy, Ethoxyethyloxy, Ethoxypropyloxy oder Propoxypropyloxy.

Reste R¹⁵ sind z.B. 2-, 3- oder 4-Fluorphenyl, 2-, 3- oder 4-Chlorphenyl, 2-, 3- oder 4-Bromphenyl, 2-, 3- oder 4-Methylphenyl, 2-, 3- oder 4-Ethylphenyl, 2-, 3- oder 4-Methoxyphenyl, 2-, 3- oder 4-Cyanophenyl, 2-, 3- oder 4-Hydroxyphenyl, 2-, 3-oder 4-Acetylphenyl, 2-, 3- oder 4-Acetylaminophenyl, 2-, 3-oder 4-Sulfamoylphenyl oder 2-, 3- oder 4-Carbamoylphenyl.

Die Reste R⁶, R¹⁰ und R¹⁴ sind ferner z.B. Methoxycarbonyl, Ethoxycarbonyl, Propoxycarbonyl, Isopropoxycarbonyl, Butoxycarbonyl, Isobutoxycarbonyl, sec-Butoxycarbonyl, tert-Butoxycarbonyl, Pentoxycarbonyl, Isopentoxycarbonyl, Neopentoxycarbonyl, tert.-Pentyloxycarbonyl, Hexyloxycarbonyl oder 2-Methylpentyloxycarbonyl.

Die Reste R⁶ sind weiterhin beispielsweise Mono- oder Dimethylsulfamoyl, Mono- oder Diethylsulfamoyl, Mono- oder Dipropylsulfamoyl, Mono- oder Dibutylsulfamoyl.

Als Reste R¹¹ kommen ferner in Betracht z.B. Aminomethyl, 2-Aminoethyl, 2- oder 3-Aminopropyl, 2- oder 4-Aminobutyl, Acetylaminomethyl, 2-Acetylaminoethyl, 2- oder 3-Acetylaminopropyl, 2- oder 4-Acetylaminobutyl, Benzoylaminomethyl, 2-Benzoylaminoethyl, 2-oder 3-Benzoylaminopropyl, 2- oder 4-Benzoylaminobutyl, 2-, 3-oder 4-Benzoylaminophenyl, 2-, 3- oder 4-Acetylaminophenyl, 2-, 3- oder 4-Methylphenyl, 2-, 3- oder 4-Methoxyphenyl, 2-, 3- oder 4-Cyanophenyl, 2-, 3- oder 4-Chlorphenyl, Phenylamino, Methylamino, Ethylamino, Propylamino, Isopropylamino, Butylamino, Isobutylamino, sec-Butylamino, tert-Butylamino, Acetylamino, Propionylamino, Butyrylamino, Isobutyrylamino, Valerylamino, Isovalerylamino, Pivaloyl, Pentylcarbonyl oder Benzoylamino.

Im folgenden werden beispielhaft Kupplungskomponenten KH aufgeführt. Als Kupplungskomponenten der Benzolreihe sind z.B. Resorcin, 2- und 4-Methylresorcin, 1,3-Phenylendiamin, 3-Aminophenol, 4-Methyl-3-aminophenol, 5-Amino-2-methylphenol, 4-Ethyl-3-aminophenol, 3-N,N-Diethylaminophenol, 2,4-Diaminotoluol, 2,4-Dihydroxybenzoesäure, 4-Methyl-2-aminophenol, 3-Acetaminophenol, 3-Amino-4-hydroxybenzolsulfonamid, o-, m- oder p-Toluidin, o-, m- oder p-Xylidin, 2,5-Dimethoxyanilin, 2-Methoxy-5-methylanilin, 3-Amino-4-methylacetanilid, 2- oder 4-Methoxyacetanilid, N-Methylanilin, N-Methyl-m-toluidin, N-Ethylanilin, N,N-Diethylanilin, E-Ethyl-m-toluidin, N-(2-Hydroxyethyl)anilin, N,N-Dihydroxyethylanilin oder N-(2-Hydroxyethyl)-m-toluidin zu nennen.

Kupplungskomponenten der Naphthalinreihe sind z.B. 1-Naphthylamin, N-Phenyl-1-naphthylamin, N-Ethyl-1-naphthylamin, N-Ethyl-2-naphthylamin, N-Phenyl-2-naphthylamin, 1-Naphthol, 2-Naphthol, 2,5-Dihydroxynaphthalin, 1,6-Dihydroxynaphthalin, 1,7-Dihydroxynaphthalin, 2,7-Dihydroxynaphthalin, 2-Hydroxynaphthalin-1-carbonsäure, 2-Hydroxynaphthalin-3-carbonsäure, 2-Hydroxynaphthalin-3-carbonsäuremethylester oder 2-Hydroxynaphthalin-3-carbonsäureamid.

Kupplungskomponenten der Chinolinreihe sind z.B. 8-Hydroxychinolin, 8-Hydroxy-2-methylchinolin, 8-Aminochinolin, 8-Amino-2-methylchinolin, 2-Hydroxychinolin, 2,4-Dihydroxychinolin zu nennen.

Als Pyridonkupplungskomponenten sind beispielsweise 1-Ethyl-2-hydroxy-4-methyl-5-carbamoylpyrid-6-on, 1-(2'-Hydroxyethyl)2-hydroxy-4-methyl-5-carbamoylpyrid-6-on, 1-Phenyl-2-hydroxy-4-methyl-5-carbamoylpyrid-6-on, 1-Ethyl-2-hydroxy-4-methyl-5-cyanopyrid-6-on, 1-Methyl-2-hydroxy-4-methyl-5-cyanopyrid-6-on, 1-Methyl-2-hydroxy-5-acetylpyrid-6-on, 1,4-Dimethyl-2-hydroxy-5-cyanopyrid-6-on, 1,4-Dimethyl-5-carbamoylpyrid-6-on, 2,6-Dihydroxy-4-ethyl-5-cyanopyridin, 2-Hydroxy-4-ethyl-5-carbamoylpyrid-6-on oder 1-Methyl-2-hydroxy-4-methylsulfonylpyrid-6-on zu nennen.

Als Kupplungskomponente der Barbitursäurereihe sind z.B. Barbitursäure, Dimethylbarbitursäure und Diethylbarbitursäure geeignet.

Als Pyrazolon-Kupplungskomponenten sind beispielsweise 3-Methyl-oder 3-(C₁-C₄-Alkoxycarbonyl)pyrazol-5-one zu nennen, die in 1-Stellung Wasserstoff, gegebenenfalls durch Methyl, Ethyl, Fluor, Chlor, Brom, Methoxy, Ethoxy, Cyano, Acetylamino, Hydroxyl, Carbamoyl oder Sulfamoyl substituiertes Phenyl tragen können. Beispielhaft seien 1-(2',5'-Dichlorphenyl)-3-methyl- pyrazol-5-on, 1-Phenylpyrazol-5-on-3-carbonsäureethylester und Pyrazol-5-on-3-carbonsäureethylester erwähnt.

Bevorzugt werden Farbstoffe der allgemeinen Formel I, in der R¹ Wasserstoff bedeutet.

Außerdem werden Farbstoffe der allgemeinen Formel I bevorzugt, in der Q¹ Wasserstoff bedeutet. Farbstoffe der Formel I, in der Q¹, Q² und Q³ unabhängig voneinander Wasserstoff oder C₁-C₄-Alkyl bedeuten, sind ebenfalls bevorzugt, insbesondere werden solche Farbstoffe bevorzugt, in denen Q¹ Wasserstoff und Q² und Q³ unabhängig voneinander C₁-C₄-Alkyl bedeuten.

Bevorzugte Reste B sind C₂-C₆-Alkylen, das durch Oxy oder C₁-C₆-Alkylimino unterbrochen sein kann. Diese Brückenglieder enthaltenden Farbstoffe werden ebenfalls bevorzugt.

Als Kupplungskomponenten KH werden Verbindungen der Formel IIa und IIb bevorzugt. Besonders bevorzugt werden Kupplungskomponenten der Formel IIa, in der R⁴ für Hydroxy, Amino, Mono- oder Dimethyl- oder -ethylamino oder Mono- oder Dihydroxyethylamino steht und R⁵ und R⁶ unabhängig voneinander für Wasserstoff, Hydroxy, Amino oder Methyl stehen.

Ebenfalls besonders bevorzugt werden Kupplungskomponenten der Formel IIb, in der R⁷ Hydroxy oder Amino bedeutet.

Besonders bevorzugt werden die folgenden Kupplungskomponenten: Resorcin, 3-Aminophenol, m-Phenylendiamin, α- und β-Naphthol, m-Toluidin und 2,5-Dimethylanilin.

Ferner werden Farbstoffe der Formel I bevorzugt, in der n die Zahl 0 bedeutet.

Zur Herstellung der Farbstoffe der Formel I kann man ein Amin der Formel III 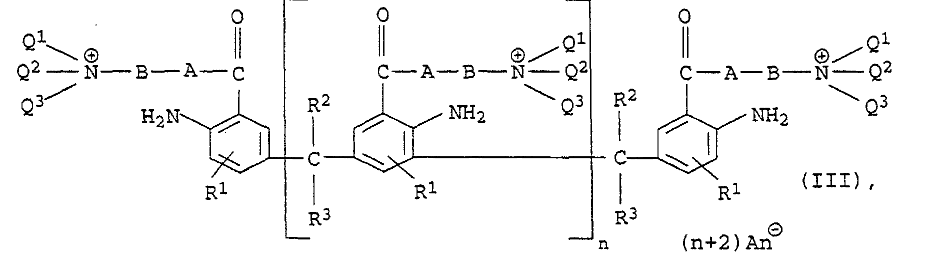 in der n, R¹, R², R³, A, B, Q¹, Q², Q³ und An^{⊖} die oben angegebene Bedeutung haben, nach bekannten Methoden diazotieren und mit einer Kupplungskomponente KH kuppeln. Bevorzugt wählt man einen pH-Wert im Bereich von 5 bis 10.

Vorteilhaft dosiert man die Diazoniumsalzlösung zur Lösung der Kupplungskomponente. Es ist generell möglich, eine einzelne Kupplungskomponente sowie Gemische mehrerer Kupplungskomponenten zu verwenden, z.B. um den Farbton abzutönen.

Durch Wahl von Kupplungskomponenten, die zweifach kuppeln können, ist es möglich durch die Azokupplungsreaktion höhermolekulare Farbstoffe zu erhalten. Diese oligomeren Farbstoffe sind erhältlich durch Azokupplung von 1 Moläquivalent Amin der Formel III auf (n+2)/2 bis n+2, vorzugsweise auf (n+2)/2 bis (n+2)/1,5, Moläquivalente einer Kupplungskomponente der Formel IIa' 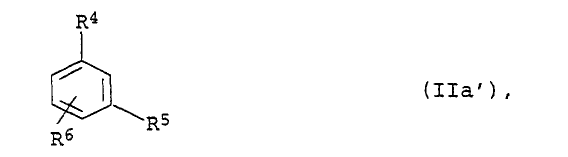 in der
- R⁴ und R⁵: unabhängig voneinander Hydroxy, Amino, Morpholino oder Mono- oder Di-(C₁-C₆)-alkylamino, deren Alkylreste gegebenenfalls mit Hydroxy, Amino, Cyano, C₁-C₆-Alkoxycarbonyl, Carbamoyl oder Mono- oder Di-(C₁-C₆)-alkylcarbamoyl substituiert sind und gegebenenfalls durch Oxy unterbrochen sind und
- R⁶: Wasserstoff, C₁-C₆-Alkoxy, dessen Alkylrest gegebenenfalls durch Oxy unterbrochen ist, Mono- oder Di-(C₁-C₆)-alkylamino, C₁-C₆-Alkoxycarbonyl, Carbamoyl, Mono- oder Di-(C₁-C₆)-alkylcarbamoyl, Sulfamoyl, Mono-oder Di-(C₁-C₆)-alkylsulfamoyl bedeuten und
wobei n der Anzahl der Wiederholungseinheiten des Amins, wie in Formel III definiert, entspricht.

Analog zu den Farbstoffen I setzt sich das entsprechende Amin III aus n+2 Aminobenzoesäurebausteinen zusammen.

Bevorzugt werden kationische Azofarbstoffe, die durch Azokupplung von 1 Moläquivalent Diamin der Formel III' (n=0) mit 1 bis 2, bevorzugt 1,5, insbesondere 1,2 Moläquivalenten einer Kupplungskomponente der Formel IIa' erhältlich sind. Besonders bevorzugt werden Azofarbstoffe, die durch den äquimolaren Einsatz von Diazo- und Kupplungskomponente erhalten werden.

Der Kupplungsschritt wird bei einem pH-Wert oberhalb 5, bevorzugt in einem Bereich von 7 bis 10 durchgeführt. Die Einstellung des gewünschten pH-Werts sowie gegebenenfalls eine Korrektur während der Kupplungsreaktion kann beispielsweise durch Zugabe von Ammonium- oder Alkalimetallbasen, z.B. Natrium- oder Kaliumhydroxyd, -carbonat oder -acetat erfolgen. Üblicherweise ist der Endpunkt der Reaktion nach spätestens 2 Stunden nach beendeter Zugabe erreicht. Der Kettenabbruch erfolgt durch Kupplung auf eine Kupplungskomponente, die kein reaktionsfähiges Ende mehr besitzt, oder durch Zersetzung der Diazoniumverbindungen.

Die vorliegende Erfindung betrifft auch die obengenannten Amine der Formel III, die in Form ihrer Ammoniumsalze dargestellt sind. Natürlich sind auch die Amine selber von den Ansprüchen mit umfaßt.

Bevorzugt werden Amine der Formel III, in der R¹ Wasserstoff bedeutet.

Ebenfalls bevorzugt werden Amine der Formel III, in der Q¹ Wasserstoff bedeutet. Ebenfalls bevorzugt werden Amine der Formel III, in der Q¹, Q² und Q³ unabhängig voneinander Wasserstoff oder C₁-C₄-Alkyl bedeuten, insbesondere solche, in denen Q¹ Wasserstoff und Q² und Q³ unabhängig voneinander C₁-C₄-Alkyl bedeuten.

Außerdem werden Amine der Formel III bevorzugt, in der B für C₂-C₆-Alkylen steht, das durch Oxy oder C₁-C₄-Alkylimino unterbrochen sein kann.

Bevorzugt werden Amine der Formel III' mit n=0.

Die neuen Amine der Formel III werden erhalten, indem man zwei Aromaten der allgemeinen Formel IV 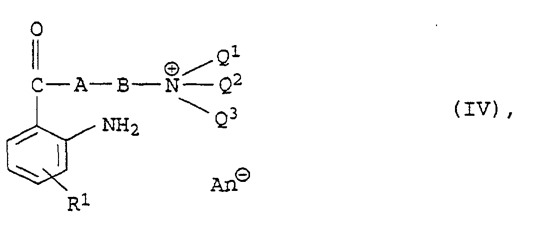 in der
R¹, A, B, Q¹, Q², Q³ und An^{⊖} die obengenannte Bedeutung haben, mit Aldehyden und Ketonen der Formel V 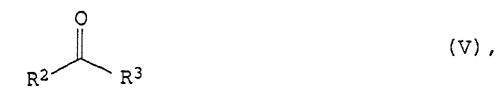 in der R² und R³ die obengenannte Bedeutung haben, im Sauren kondensiert.

Geeignete Aldehyde und Ketone sind z.B. Formaldehyd, Acetaldehyd, Propionaldehyd, Butyraldehyd, Aceton, Methylethylketon und Cyclohexanon. Bevorzugt wird Formaldehyd eingesetzt.

Solche Kondensationen am aromatischen Kern sind generell bekannt. Die beiden Ausgangsverbindungen werden für gewöhnlich bei pH 0 bis 5 in wäßriger Lösung z.B. im Salzsauren oder Schwefelsäuresauren umgesetzt. In der Regel wählt man Temperaturen von 20 bis 70°C. Abhängig von der Wahl der Temperatur und Verdünnung ist die Reaktion üblicherweise nach 1 bis 6 Stunden beendet.

Die Aromaten der Formel (IV) können am Benzol sowohl in para- als auch in ortho-Position zur Aminogruppe kondensiert werden, wobei die para-Position üblicherweise die reaktivere ist.

Demzufolge erhält man durch Wahl des Molverhältnisses der Ausgangsverbindungen neben dem dimeren Amin III mit n=0 im zunehmenden Maße auch oligomere Amine III mit n > 0, wenn das Molverhältnis Amin(III)/Aldehyd,Keton(V) 2/1 übersteigt.

Ebenso kann ein zwischenzeitlicher Überschuß an Aldehyd/Keton(V), wie er beispielsweise bei rascher Zudosierung des Aldehyds/Ketons oder Vorlage des Aldehyds/Ketons und Zudosierung des Amins auftritt, zu einem höheren Gehalt an Oligomeren führen.

Beabsichtigt man die Herstellung der Dimeren, wird man demnach vorzugsweise das Amin vorlegen und das Aldehyd/Keton zudosieren. Eine Reinigung des Dimeren ist durch beispielsweise Kristallisation möglich.

Bevorzugt werden jedoch auch in der Farbstoffsynthese die Gemische der dimeren und oligomeren Amine verwendet, die zu Farbstoffgemischen mit vorteilhaften anwendungstechnischen Eigenschaften führen, bei denen sich die einzelnen Farbstoffe lediglich in ihrer Anzahl an Wiederholungseinheiten unterscheiden.

Wählt man dagegen im oben beschriebenen Kondensationsschritt ein Molverhältnis Amin(III)/Aldehyd,Keton(V) von kleiner 2/1, so erhält man bei Umsetzung des Produktgemisches nach Diazotierung und Kupplung Farbstoffmischungen, die neben einem oder mehreren Farbstoffen der Formel I auch einen oder mehrere Farbstoffe der Formel (VI) 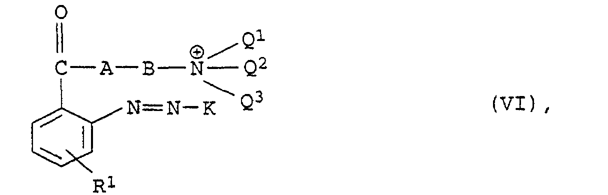 in der R¹, K, A, B, Q¹, Q², Q³ und An⊖ die obengenannte Bedeutung haben enthalten. So erhaltene Farbstoffmischungen sind ebenfalls bevorzugt.

Die erfindungsgemäßen basischen Azofarbstoffe der Formel I können für sich alleine, in Gemischen untereinander und zusammen mit anderen kationischen oder anionischen Verbindungen in Form ihrer Lösungen oder in Form von Pulvern oder Granulaten angewendet werden. Sie eignen sich vorteilhaft zum Färben oder Bedrucken von polymerem Material, insbesondere von Papierstoffen wie Papier und Karton, aber auch von Cellulose, Baumwolle, Leder, Bastfasern, Hanf, Flachs, Sisal, Jute, Kokos, Stroh oder anionisch modifizierten Fasern sowie in Aufzeichnungsflüssigkeiten wie Tinten, insbesondere für den Inkjet-Druck oder Druckfarben.

Bei der Herstellung von Farbstoffpräparationen, enthaltend die neuen Farbstoffe der Formel I, ist die Anwendung von Polymeren, wie Polyacrylsäuren, Polyacrylsäurederivaten, Polyvinylaminen, Polyvinylamiden, Polyvinylacetaten, Polyvinylalkoholen, Polyvinylpyrrolidonen, Polysiloxanen oder Copolymeren der jeweiligen Monomeren hervorzuheben. Desgleichen können Oligomere des Ethylenimins, Ethylenoxids oder Propylenoxids oder Derivate dieser Oligomeren zur Anwendung kommen.

Die Farbstoffe können vorzugsweise bei der Herstellung von in der Masse gefärbtem, geleimtem und ungeleimtem Papier verwendet werden. Sie können ebenfalls zum Färben von Papier nach dem Tauchverfahren angewendet werden.

Das Färben von Papier, Leder oder Cellulose erfolgt nach an sich bekannten Methoden.

Die neuen Farbstoffe oder ihre Präparationen färben das Abwasser bei der Papierherstellung praktisch gar nicht oder nur wenig an, was für die Reinhaltung der Gewässer besonders günstig ist. Sie sind hoch substantiv, melieren nicht, wenn sie auf Papier gefärbt sind und sind weitgehend pH-unempfindlich. Die Färbungen auf Papier zeichnen sich durch eine gute Lichtechtheit aus. Nach längerem Belichten ändert sich die Nuance Ton-in-Ton.

Die gefärbten Papiere, die eine gute Bleichbarkeit aufweisen, sind naßecht, nicht nur gegen Wasser, sondern ebenfalls gegen Milch, Seifenwasser, Natriumchloridlösungen, Fruchtsäfte oder gesüßte Mineralwasser und wegen ihrer guten Alkoholechtheit auch gegen alkoholische Getränke beständig.

Mit den neuen Farbstoffen kann man auch Polyacrylnitriltextilien oder durch anionische Gruppen modifizierte Polyamid- oder Polyestertextilien färben, foulardieren oder bedrucken.

Die folgenden Beispiele sollen die Erfindung näher erläutern.

### Beispiel A

208,2 g (1 mol) 2-Amino-(2-dimethylamino-ethyl)-benzoat wurden in einer Mischung aus 900 ml Wasser und 70 ml konz. Schwefelsäure gelöst. Zu dieser Lösung gab man bei 50°C innerhalb von zwei Stunden 41,1 g (0,5 mol) Formaldehyd-Lösung (36,5 gew.-%ig) zu und rührte nach beendeter Zugabe noch weitere 4 Stunden bei 50°C nach. Anschließend kühlte man auf auf 0°C ab und stellte mit 10 gew.-%iger Sodalösung einen pH-Wert von 8,0 ein. Die Lösung wurde danach mit jeweils 300 ml Methyl-tert.-butylether 6mal extrahiert und die vereinigten organischen Phasen mit Natriumsulfat getrocknet. Nach Abdestillieren des Ethers im Vakuum erhielt man 138,1 g Rohprodukt als nahezu farbloses Öl. Umkristallisation aus Toluol ergab 94,1 g 6,6'-Diamino-bis-(2-dimethylamino-ethyl) -3,3'-methylen-bis-benzoat als kristallines Reinprodukt. 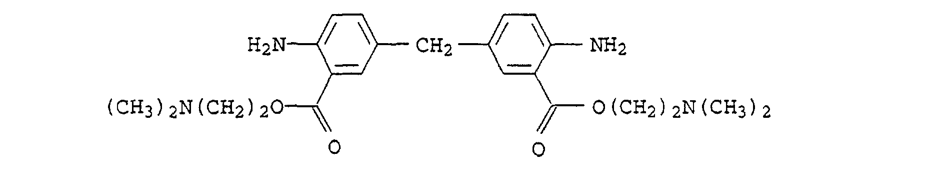 Schmp.: 87-89 °C
¹H-NMR (CDCl₃): δ = 2.32 (s, 12H, CH₃), 2.68 (t, 4H, CH₂), 3.75 (s, 2H, CH₂), 4.35 (t, 4H, CH₂), 5.60 (s, 4H, NH₂), 6.60 (d, 2H, Hₐᵣₒₘ), 7.05 (d, 2H, Hₐᵣₒₘ), 7.66 (s, 2H, Hₐᵣₒₘ).

### Beispiel B

25,2 g (0,1 mol) 2-Amino-(2-(2-dimethylamino-ethoxy)-ethyl)-benzoat wurden in einer Mischung aus 50 ml Wasser und 7 ml konz. Schwefelsäure gelöst und anschließend bei 50°C 4,1 g (0,05 mol) einer 36,5 gew.-%igen Formaldehyd-Lösung zugetropft. Nach beendeter Zugabe rührte man noch 4 Stunden bei 50°C nach, kühlte dann auf 0°C ab und stellte mit 10 gew.-%iger Sodalösung einen pH-Wert von 8,0 ein. Danach extrahierte man die Emulsion dreimal mit jeweils 100 ml Essigester, vereinigt die organischen Phasen und trocknet sie mit Natriumsulfat. Nach Abdestillieren des Essigesters im Vakuum erhielt man 15,1 g 6,6'-Diamino-bis-(2-(2-di-methylamino-ethoxy)-ethyl)-3,3'-methylen-bis-benzoat als schwach gelbes, zähes Öl. 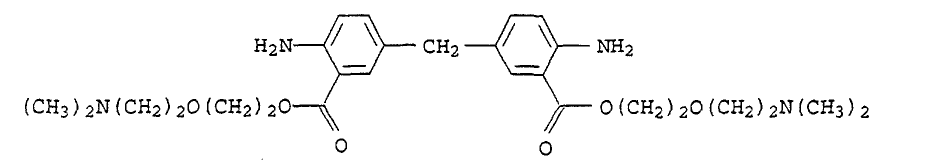 ¹H-NMR (CDCl₃): δ = 2.25 (s, 12H, CH₃), 2.53 (t, 4H, CH₂), 3.63 (t, 4H, CH₂), 3.73 (s, 2H, CH₂), 3.80 (t, 4H, CH₂), 4.40 (t, 4H, CH₂), 5.60 (s, 4H, NH₂), 6.56 (d, 2H, Hₐᵣₒₘ), 7.03 (d, 2H, Hₐᵣₒₘ), 7.70 (s, 2H, Hₐᵣₒₘ).

### Beispiel C

20,7 g 2-Amino-N-(2-dimethylamino-ethyl)-benzamid wurden in einer Mischung aus 90 ml Wasser und 7 ml konz. Schwefelsäure gelöst, dann auf 50°C erwärmt und bei dieser Temperatur innerhalb von 15 Minuten 4,1 g einer 36,5 gew.-%igen Formaldehydlösung zutropft. Anschließend rührte man noch 4 Stunden bei 50°C nach, kühlte auf Raumtemperatur ab und stellte die Lösung mit 10 gew.-%iger Natronlauge auf pH 9. Das Produkt schied sich als Öl ab und wurde im Scheidetrichter von der Wasserphase abgetrennt. Man erhielt nach dem Trocknen im Vakuum 21,5 g 6,6'-Diamino-N,N'-bis-(2-dimethylamino-ethyl)-3,3'-methylen-bisbenzamid als glasartig erstarrende Substanz. 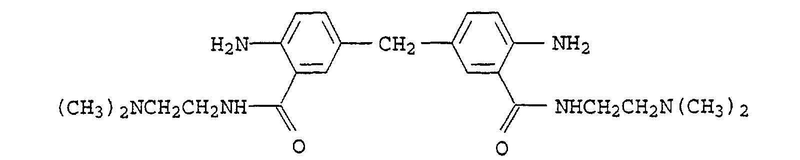 1H-NMR (CDCl₃): δ = 2.22 (s, 12H, CH₃), 2.46 (t, 4H, CH₂), 3.44 (t, 4H, CH₂), 3.71 (s, 2H, CH₂), 5.42 (s, 4H, NH₂), 6.57 (d, 2H, Hₐᵣₒₘ), 6.97 (d, 2H, Hₐᵣₒₘ) , 7.15 (s, 2H, Hₐᵣₒₘ).

### Beispiel 1

4,3 g (0,01 mol) 6,6'-Diamino-bis-(2-dimethylamino-ethyl)-3,3'-methylen-bis-benzoat (gemäß Beispiel A) wurden in 50 ml Wasser und 6 ml konz. Salzsäure gelöst und zu der Lösung bei 0 bis 5°C langsam 6,6 ml Natriumnitritlösung (23 %ig) zugegeben. Anschließend rührte man noch 45 Minuten bei 0 bis 5°C nach und vernichtete dann überschüssiges Nitrit durch Zugabe von Amidosulfonsäure. Die Diazoniumsalzlösung wurde dann zu einer Lösung von 2,2 g Resorcin in 200 ml Wasser getropft. Gleichzeitig wurde durch Zugabe von 10 gew-%iger Natronlauge ein pH-Wert von 9,5 bis 10,0 eingestellt und die Temperatur durch Eiszugabe bei 0°C gehalten. Nach beendeter Zugabe rührte man noch 10 Minuten bei 0°C und pH 10 nach und stellte dann mit konz. Salzsäure bei 0°C einen pH-Wert von 7,2 ein. Dann ließ man den Ansatz über Nacht auf Raumtemperatur erwärmen. Der entstandene orangerote Niederschlag wurde abgesaugt, mit Wasser gewaschen und bei 60°C im Vakuum getrocknet. Man erhielt 6,2 g eines Farbstoffs der Formel 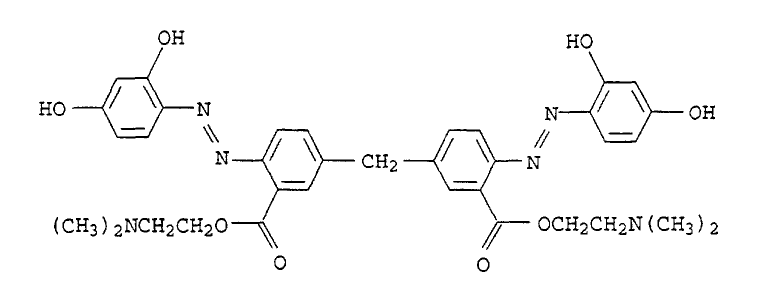 λₘₐₓ (verd. Essigsäure): 402 nm

Analog zu Beispiel 1 können aus 6,6'-Diamino-bis-(2-dimethylamino-ethyl)-3,3'-methylen-bis-benzoat als Diazokomponente und den in der nachfolgenden Tabelle 1 aufgeführten Kupplungskomponenten Farbstoffe mit guten Lichtechtheiten hergestellt werden:

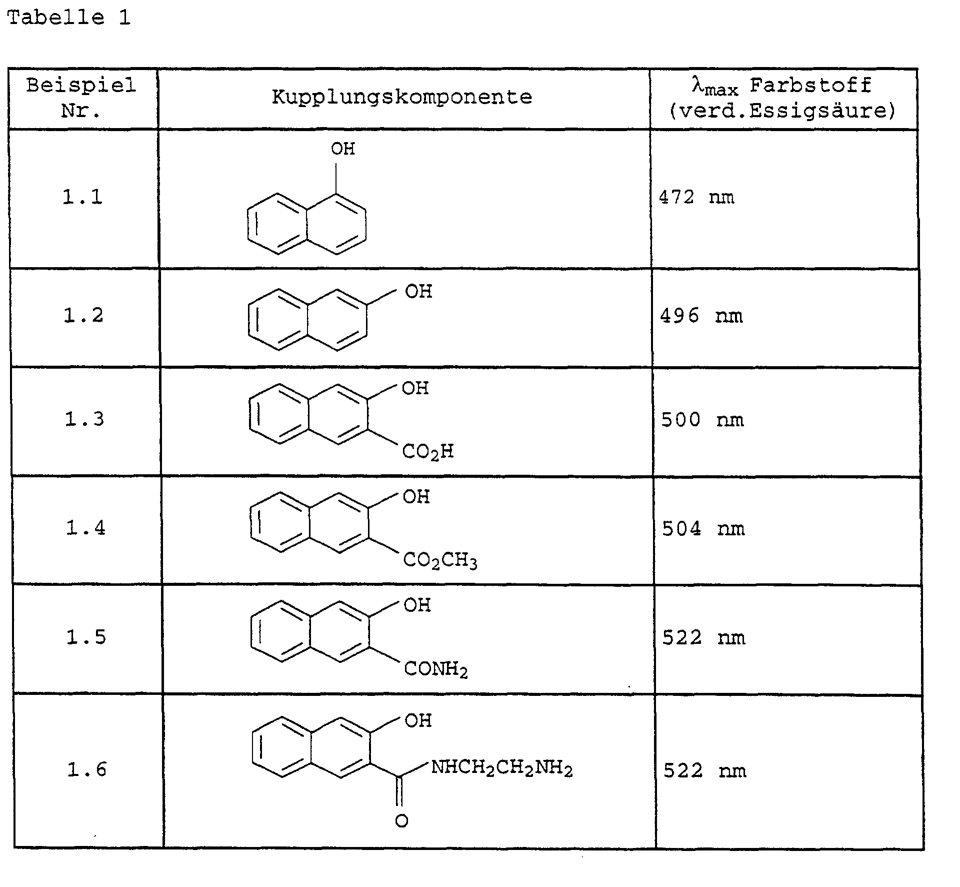

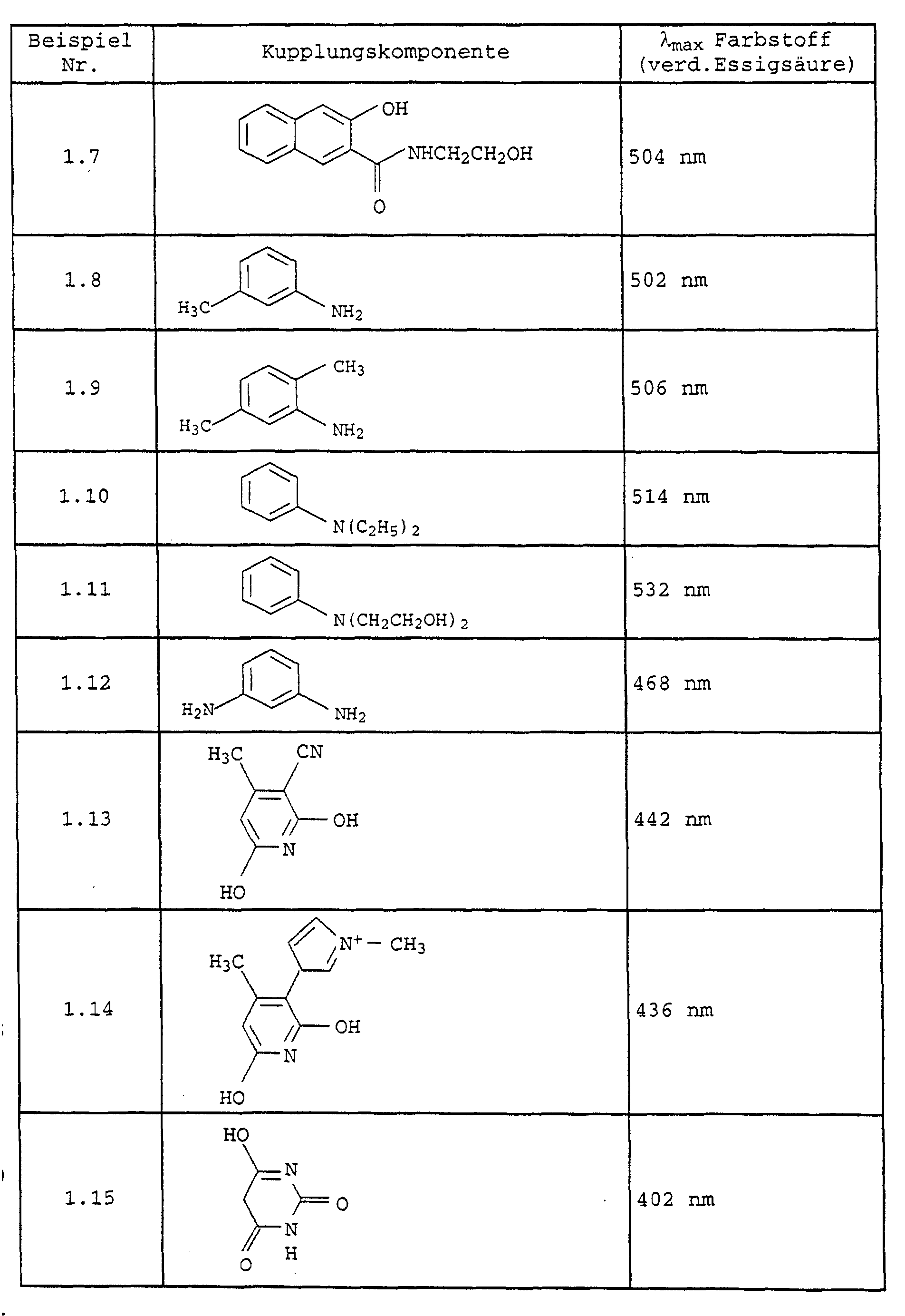

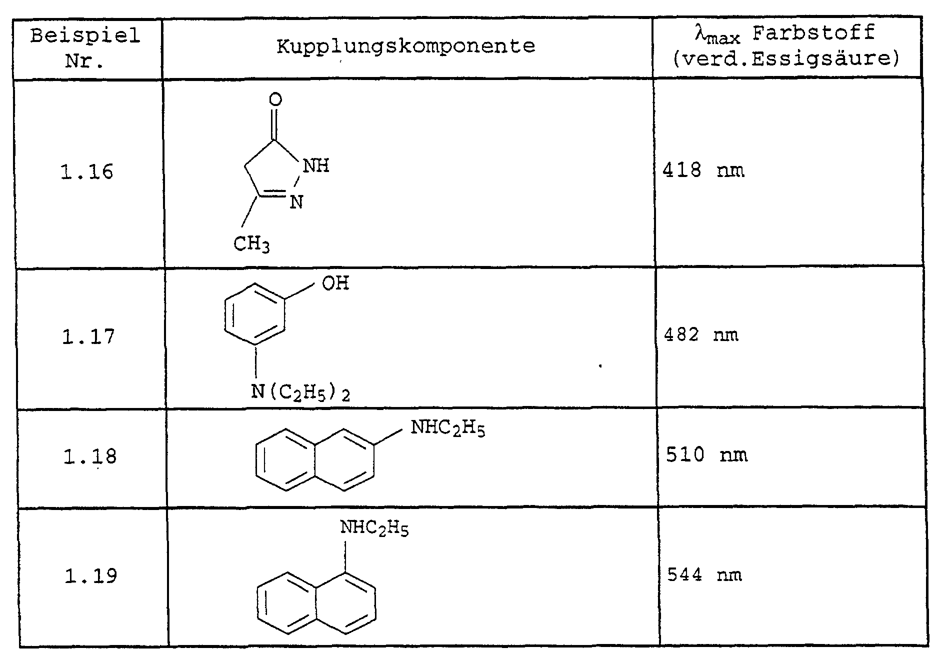

Ebenso können analog zu Beispiel 1 durch Diazotierung und Kupplung von 6,6'-Diamino-bis-(2-(2-dimethylamino-ethoxy)-ethyl)-3,3'-methylen-bis-benzoat (Beispiel B) oder 6,6'-Diamino-N,N'-bis-(2-dimethylamino-ethyl)-3,3'-methylen-bis-benzamid (Beispiel C) Farbstoffe mit guten anwendungstechnischen Eigenschaften erhalten werden.

### Beispiel 2

20,8 g (0,1 mol) 2-Amino-(2-dimethylamino-ethyl)-benzoat wurden in einer Mischung aus 90 ml Wasser und 7 ml konz. Schwefelsäure gelöst. Zu dieser Lösung gab man bei 50°C innerhalb 30 Minuten 4,1 g (0,05 mol) 36,5 gew.-%ige Formaldehydlösung und rührte noch weitere 4 Stunden bei 50°C nach. Anschließend kühlte man auf 0°C ab und gab erst 20,4 g (11,5 ml) konz. Schwefelsäure und dann bei 0 bis 5°C 30 ml einer 23 gew.-%igen Natriumnitritlösung zu. Man rührte noch eine halbe Stunde bei 0 bis 5°C nach und vernichtete dann überschüssiges Nitrit durch Zugabe von Amidosulfonsäure.

Die Diazoniumsalzlösung wurde zu einer Lösung von 12,1 g Resorcin in 150 ml Wasser getropft. Hierbei wurde durch gleichzeitige Zudosierung von 10 gew.-%iger Natronlauge ein pH-Wert von 9,0 eingestellt, wobei die Temperatur bei 0°C gehalten wurde. Nach beendeter Zugabe rührte man noch 10 Minuten bei 0°C und pH 9,0 nach und stellte dann mit 10 gew.-%iger Schwefelsäure bei 0°C einen pH-Wert von 7,2 ein. Dann ließ man den Ansatz über Nacht auf Raumtemperatur erwärmen und saugte anschließend den entstandenen rotbraunen Niederschlag ab. Der Niederschlag wurde gewaschen und bei 60°C im Vakuum getrocknet. Man erhielt 32,6 g einer Farbstoffmischung mit dem in Beispiel 1 beschriebenen Farbstoff als Hauptkomponente und trimeren und oligomeren Derivaten (siehe Beispiel 3) als Nebenkomponenten.
λₘₐₓ (verd. Essigsäure): 400 nm

### Beispiel 3

20,8 g (0,1 mol) 2-Amino-(2-dimethylamino-ethyl)-benzoat wurden in einer Mischung aus 90 ml Wasser und 7 ml konz. Schwefelsäure gelöst. Anschließend gab man bei 50°C in einer halben Stunde 9,0 g (0,1 mol) Formaldehyd-Lösung (36,5 gew.-%ig) zu und rührte noch weitere 4 Stunden bei 50°C nach. Anschließend kühlte man auf auf 0°C ab und gab erst 20,4 g (11,5 ml) konz. Schwefelsäure und dann bei 0 bis 5°C 30 ml einer 23 gew.-%igen Natriumnitritlösung zu. Man rührte noch eine halbe Stunde bei 0 bis 5°C nach und vernichtete dann überschüssiges Nitrit durch Zugabe von Amidosulfonsäure.

Die Diazoniumsalzlösung wurde zu einer Lösung von 12,1 g Resorcin in 150 ml Wasser getropft. Hierbei wurde durch gleichzeitige Zudosierung von 10 gew.-%iger Natronlauge ein pH-Wert von 9,0 eingestellt und die Temperatur durch Eiszugabe bei 0°C gehalten. Nach beendeter Zugabe rührt man noch 10 Minuten bei 0°C und pH 9,0 nach und stellte dann mit 10 gew.-%iger Schwefelsäure bei 0°C einen pH-Wert von 7,2 ein. Dann ließ man den Ansatz über Nacht auf Raumtemperatur kommen, saugte den entstandenen rotenbraunen Niederschlag ab, wusch ihn mit Wasser und trocknete anschließend bei 60°C im Vakuum. Man erhielt 31,5 g eines Gemisches oligomerer Farbstoffe der Formel 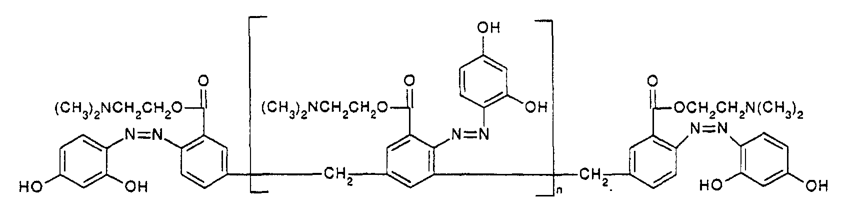 λₘₐₓ (verd. Essigsäure): 404 nm

Analog zum Beispiel 3 können die in Tabelle 2 aufgeführten Farbstoffe hergestellt werden.

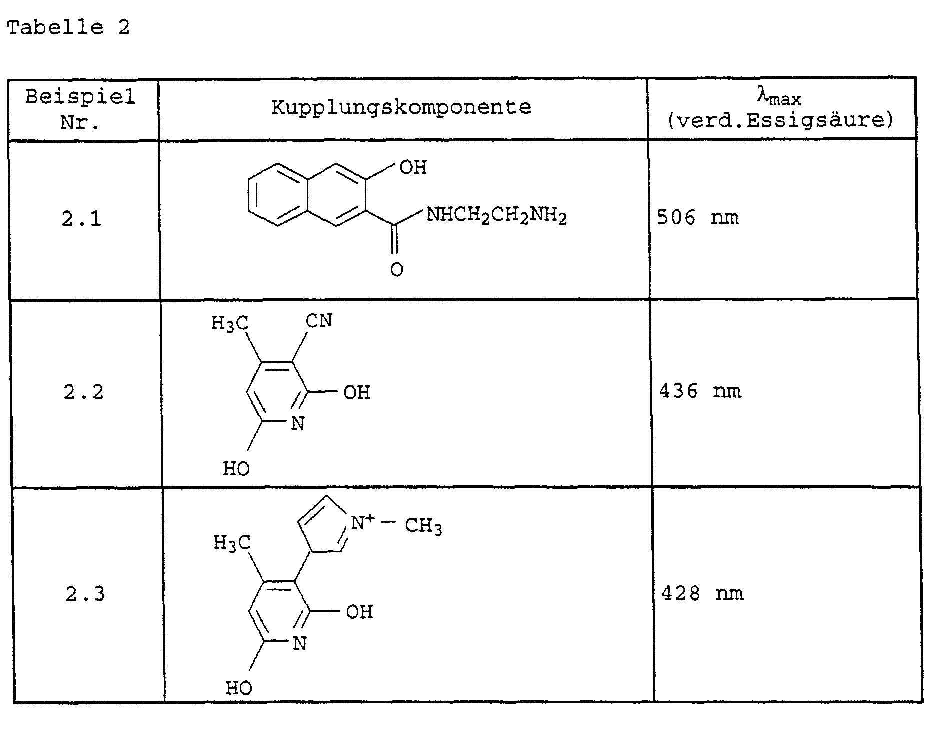

### Beispiel 4

Analog zu Beispiel 2 wurden 20,8 g (0,1 mol) 2-Amino-(2-dimethylamino-ethyl)-benzoat mit 4,1 g (0,05 mol) 36,5 gew.-%ige Formaldehydlösung umgesetzt, das Reaktionsprodukt diazotiert und auf 6,1 g (0,055 mol) Resorcin gekuppelt. Man erhielt 27,5 g einer oligomeren Farbstoffmischung, deren Absorptionsmaximum λₘₐₓ (verd. Essigsäure) sich bei 432 nm befindet.

### Beispiel 5

20,8 g 2-Amino-(2-dimethylamino-ethyl)-benzoat wurden in einer Mischung aus 90 ml Wasser und 7 ml konz. Schwefelsäure gelöst. Anschließend gab man bei 50°C in einer halben Stunde 2,0 g 36,5 gew-%ige Formaldehyd-Lösung zu und rührte noch weitere 4 Stunden bei 50°C nach. Anschließend kühlte man auf auf 0°C ab und gab erst 20,4 g (11,5 ml) konz. Schwefelsäure und dann bei 0 bis 5°C 30 ml einer 23 gew.-%igen Natriumnitritlösung zu. Man rührte noch eine halbe Stunde bei 0 bis 5°C nach und vernichtete dann überschüssiges Nitrit durch Zugabe von Amidosulfonsäure.

Die Diazoniumsalzlösung wurde zu einer Lösung von 12,1 g Resorcin in 150 ml Wasser getropft. Hierbei wurde durch gleichzeitiges Zudosieren von 10 gew.-%iger Natronlauge ein pH-Wert von 9,0 eingestellt und die Temperatur durch Eiszugabe bei 0°C gehalten. Nach beendeter Zugabe rührte man noch 10 Minuten bei 0°C und pH 9 nach und stellte dann mit 10 gew.-%iger Schwefelsäure bei 0°C einen pH-Wert von 7,2 ein. Dann ließ man den Ansatz über Nacht auf Raumtemperatur kommen, saugte den entstandenen orangen Niederschlag ab, wusch ihn mit Wasser und trocknete anschließend bei 60°C im Vakuum. Man erhielt 33,5 g einer Farbstoffmischung, bestehend aus den Farbstoffen der Formeln 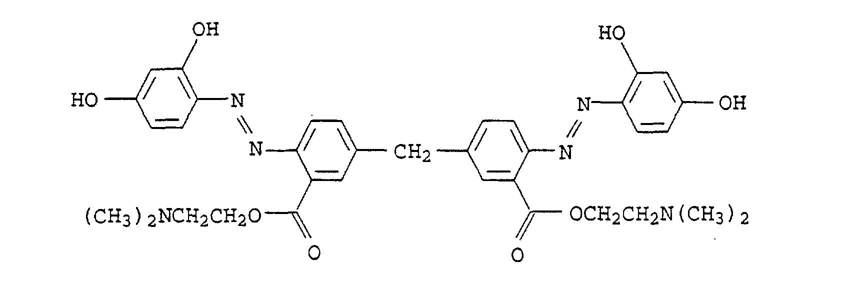 und 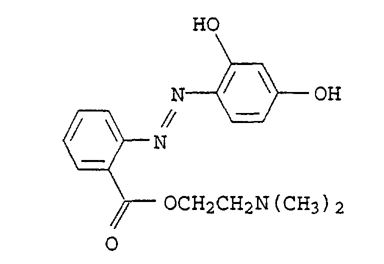 sowie trimeren und tetrameren Derivaten, wie in Beispiel 3 beschrieben mit n = 1 und 2, als Nebenkomponenten. Die Farbstoffmischung weist eine Absorptionsmaximum bei λₘₐₓ (verd. Essigsäure): 396 nm auf.

## Patentansprüche

1. Kationische Azofarbstoffe der allgemeinen Formel I 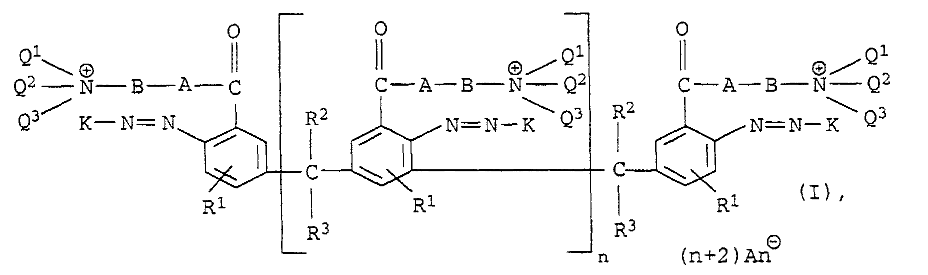 in der
n eine Zahl von 0 bis 8,
R¹ Wasserstoff, Nitro, Halogen, C₁-C₆-Alkyl,
R² und R³ unabhängig voneinander Wasserstoff, C₁-C₆-Alkyl oder zusammen mit dem sie verbindenden Kohlenstoffatom einen Cyclopentyl- oder Cyclohexylrest,
K den Rest einer Kupplungskomponente,
A Oxy, Imino oder gegebenenfalls substituiertes C₁-C₆-Alkylimino,
B C₁-C₆-Alkylen, das durch 1 oder 2 nichtbenachbarte Oxy-, Imino-, gegebenenfalls substituierte C₁-C₆-Alkyliminogruppen und einem Rest 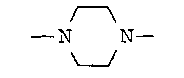 unterbrochen sein kann,
Q¹, Q² und Q³ unabhängig voneinander Wasserstoff, C₁-C₁₀-Alkyl, das durch 1 bis 4 nichtbenachbarte Oxy-, Imino- und gegebenenfalls substituierte C₁-C₄-Alkyliminogruppen unterbrochen sein kann und das mit Hydroxy, Halogen oder Phenyl substituiert sein kann und Q¹ und Q² zusammen mit dem sie verbindenden Stickstoffatom einen 5- oder 6-gliedrigen heterocyclischen Rest, der gegebenenfalls Stickstoff oder Sauerstoff als weiteres Heteroatom aufweist, und
An^{⊖} das Äquivalent eines Anions bedeuten.

2. Kationische Azofarbstoffe nach Anspruch 1, in denen K den Rest einer Kupplungskomponente aus der Benzol-, Naphthalin-, Chinolin-, Pyridon-, Barbitur- oder Pyrazolonreihe bedeutet.

3. Kationische Azofarbstoffe nach Anspruch 1 oder 2, in denen B C₂-C₆-Alkylen, das durch Oxy oder C₁-C₆-Alkylimino unterbrochen sein kann, bedeutet.

4. Kationische Azofarbstoffe, erhältlich durch Azokupplung von 1 Moläquivalent Amin der Formel III 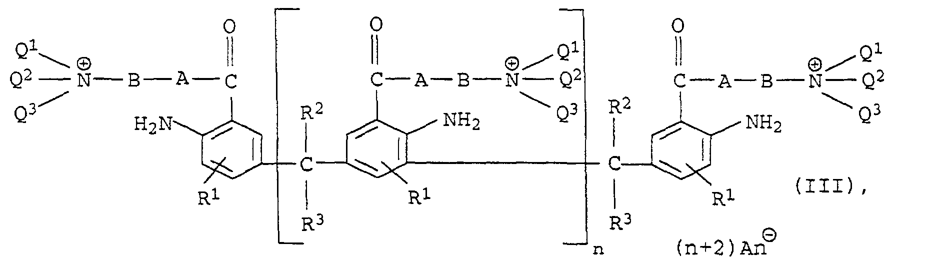 in der
n, R¹, R², R³, A, B, Q¹, Q², Q³ und An⊖ die in Anspruch 1 angegebene Bedeutung haben,
auf (n+2)/2 bis n+2 Moläquivalente einer Kupplungskomponente der Formel IIa' 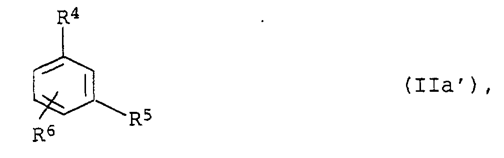 in der
R⁴ und R⁵ unabhängig voneinander Hydroxy, Amino, Morpholino oder Mono- oder Di-(C₁-C₆)-alkylamino, deren Alkylreste gegebenenfalls mit Hydroxy, Amino, Cyano, C₁-C₆-Alkoxycarbonyl, Carbamoyl oder Mono- oder Di-(C₁-C₆)-alkylcarbamoyl substituiert sind und gegebenenfalls durch Oxy unterbrochen sind und
R⁶ Wasserstoff, C₁-C₆-Alkoxy, dessen Alkylrest gegebenenfalls durch Oxy unterbrochen ist, Mono-oder Di-(C₁-C₆)-alkylamino, C₁-C₆-Alkoxycarbonyl, Carbamoyl, Mono- oder Di-(C₁-C₆)-alkylcarbamoyl, Sulfamoyl, Mono- oder Di-(C₁-C₆)-alkylsulfamoyl bedeuten.

5. Kationische Azofarbstoffe, erhältlich durch Azokupplung von 1 Moläquivalent Diamin der Formel III' (n=0) mit 1 bis 2 Moläquivalenten einer Kupplungskomponente der Formel IIa'.

6. Verfahren zur Herstellung der kationischen Azofarbstoffe gemäß den Ansprüchen 4 und 5, indem man 1 Moläquivalent Amin auf (n+2)/2 bis n+2 Moläquivalente einer Kupplungskomponente der Formel IIa' kuppelt.

7. Amine der allgemeinen Formel III 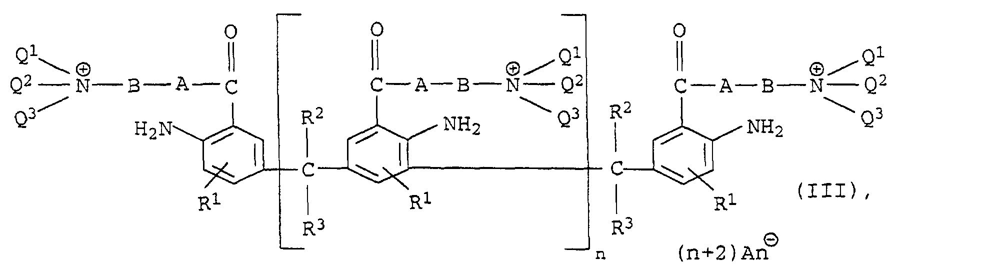 in der
n, R¹, R², R³, A, B, Q¹, Q², Q³ und An^{⊖} die in Anspruch 1 angegebene Bedeutung haben.

8. Farbstoffmischungen, enthaltend mehrere Farbstoffe der Formel I gemäß den Ansprüchen 1 bis 3, die sich in ihrer Anzahl an Wiederholungseinheiten unterscheiden.

9. Farbstoffmischungen, enthaltend einen oder mehrere Farbstoffe der Formel I gemäß den Ansprüchen 1 bis 3 sowie einen oder mehrere Farbstoffe der Formel VI 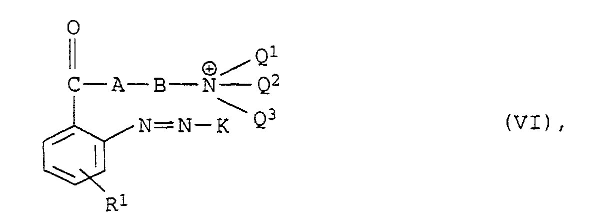 in der R¹, K, A, B, Q¹, Q², Q³ und An^{⊖} die in Anspruch 1 angegebene Bedeutung haben.

10. Verwendung der kationischen Azofarbstoffe und ihrer Mischungen gemäß den Ansprüchen 1 bis 5 und 8 und 9 zum Färben oder Bedrucken von polymerem Material.

## Claims

1. A cationic azo dye of the formula I 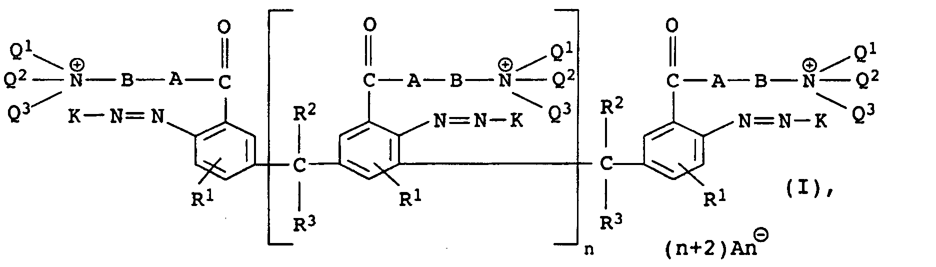 where
n is a number from 0 to 8,
R¹ is hydrogen, nitro, halogen or C₁-C₆-alkyl,
R² and R³ are independently hydrogen or C₁-C₆-alkyl or together with the carbon atom linking them are a cyclopentyl or cyclohexyl radical,
K is the radical of a coupling component,
A is oxy, imino or unsubstituted or substituted C₁-C₆-alkylimino,
B is C₁-C₆-alkylene which can be interrupted by 1 or 2 nonadjacent oxy, imino or unsubstituted or substituted C₁-C₆-alkylimino groups and a radical 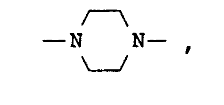
Q¹, Q² and Q³ independently are hydrogen, C₁-C₁₀-alkyl which can be interrupted by from 1 to 4 nonadjacent oxy, imino and unsubstituted or substituted C₁-C₄-alkylimino groups and can be substituted by hydroxyl, halogen or phenyl, and Q¹ and Q² together with the nitrogen atom linking them are a 5- or 6-membered heterocyclic radical with or without nitrogen or oxygen as a further heteroatom, and
An^{⊖} is the equivalent of an anion.

2. A dye as claimed in claim 1, wherein K is the radical of a coupling component from the benzene, naphthalene, quinoline, pyridone, barbituric acid or pyrazolone series.

3. A dye as claimed in claim 1 or 2, wherein B is C₂-C₆-alkylene which can be interrupted by oxy or C₁-C₆-alkylimino.

4. A cationic azo dye obtainable by azo-coupling 1 molar equivalent of amine of the formula III 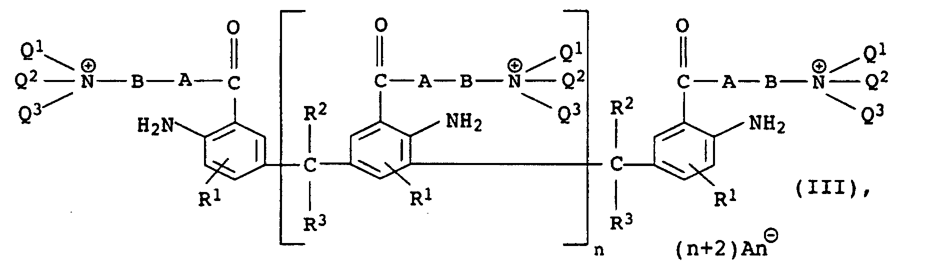 where
n, R¹, R², R³, A, B, Q¹, Q², Q³ and An^{⊖} are as defined in claim 1,
to (n+2)/2 to n+2 molar equivalents of a coupling component of the formula IIa' 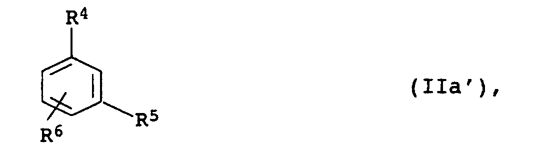
where
R⁴ and R⁵ independently of one another are hydroxyl, amino, morpholino or mono- or di-(C₁-C₆)-alkylamino whose alkyl radicals are unsubstituted or substituted by hydroxyl, amino, cyano, C₁-C₆-alkoxycarbonyl, carbamoyl or mono- or di-(C₁-C₆)-alkylcarbamoyl and uninterrupted or interrupted by oxy,
R⁶ is hydrogen or C₁-C₆-alkoxy whose alkyl radical is uninterrupted or interrupted by oxy, or is mono-or di-(C₁-C₆)-alkylamino, C₁-C₆-alkoxycarbonyl, carbamoyl, mono- or di-(C₁-C₆-alkyl)carbamoyl, sulfamoyl, mono- or di-(C₁-C₆-alkyl)sulfamoyl.

5. A cationic azo dye obtainable by azo-coupling 1 molar equivalent of diamine of the formula III' (n=0) to from 1 to 2 molar equivalents of a coupling component of the formula IIa'.

6. A process for preparing a dye as claimed in claims 4 and 5 by coupling 1 molar equivalent of amine to (n+2)/2 to n+2 molar equivalents of a coupling component of the formula IIa'.

7. An amine of the formula III 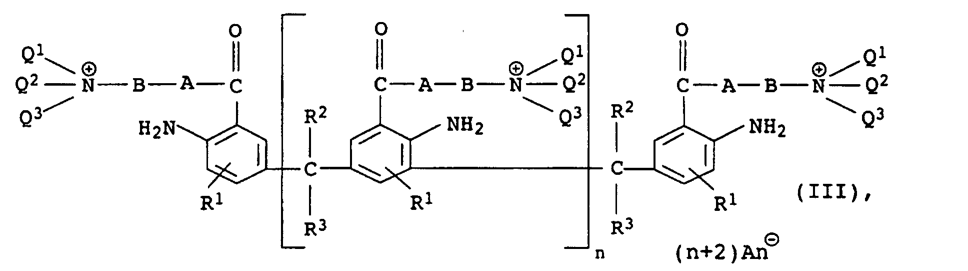 where
n, R¹, R², R³, A, B, Q¹, Q², Q³ and An^{⊖} are as defined in claim 1.

8. A dye mixture comprising two or more dyes of the formula I as claimed in claims 1 to 3 which differ in their number of repeating units.

9. A dye mixture comprising one or more dyes of the formula I as claimed in claims 1 to 3 and also one or more dyes of the formula VI 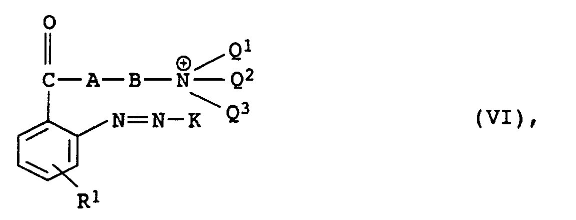 where R¹, K, A, B, Q¹, Q², Q³ and An^{⊖} are as defined in claim 1.

10. The use of a cationic azo dye or mixture of such dyes as claimed in claims 1 to 5 and 8 and 9 for dyeing or printing polymeric material.

## Revendications

1. Colorants azoïques cationiques de formule générale I 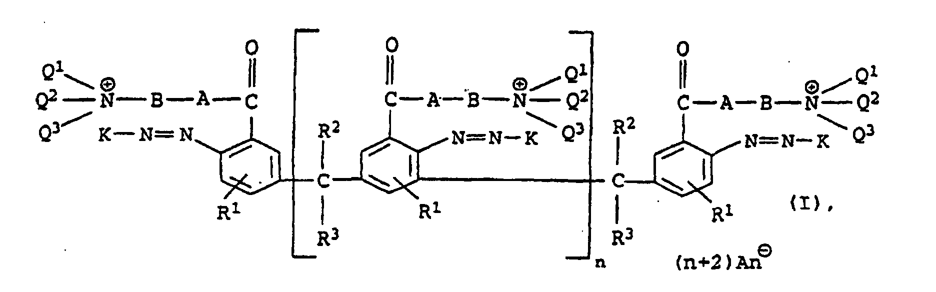 dans laquelle
n représente un nombre de 0 à 8,
R¹ représente un atome d'hydrogène, un groupe nitro, un atome d'halogène, un groupe alkyle en C₁ à C₆
R² et R³ représentent, indépendamment l'un de l'autre, un atome d'hydrogène, un groupe alkyle en C₁ à C₆ ou, ensemble avec l'atome de carbone qui les lie, un résidu cyclopentyle ou cyclohexyle,
K représente le résidu d'un composant de copulation,
A représente un groupe oxy, imino ou éventuellement un groupe alkylimino en C₁ à C₆ substitué,
B représente un groupe alkylène en C₁ à C₆, qui peut être interrompu par un ou deux groupes oxy, imino ou alkylimino en C₁ à C₆ éventuellement substitués et un résidu 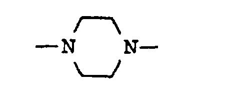
Q¹, Q² et Q³ représentent, indépendamment les uns des autres, un atome d'hydrogène, un groupe alkyle en C₁ à C₁₀, qui peut être interrompu par 1 à 4 groupes non vicinaux oxy, imino ou alkylimino en C₁ à C₄ éventuellement substitués et qui peut être substitué par un groupe hydroxy, halogéno ou phényle et Q¹ et Q² ensemble avec l'atome d'azote qui les lie, un résidu hétérocyclique à 5 ou 6 maillons, qui comprend éventuellement un atome d'oxygène ou d'azote comme hétéroatome supplémentaire, et
An^{⊖} représente l'équivalent d'un anion.

2. Colorants azoïques cationiques selon la revendication 1, dans lesquels K représente le résidu d'un composant de copulation provenant de la série des benzènes, naphtalènes, quinoléines, pyridones, barbituriques ou pyrazolones.

3. Colorants azoïques cationiques selon la revendication 1 ou 2, dans lesquels B représente un groupe alkylène en C₂ à C₆, qui peut être interrompu par un groupe oxy ou alkylimino en C₁ à C₆.

4. Colorants azoïques cationiques, pouvant être obtenus par copulation azoïque d'un équivalent molaire d'une amine de formule III 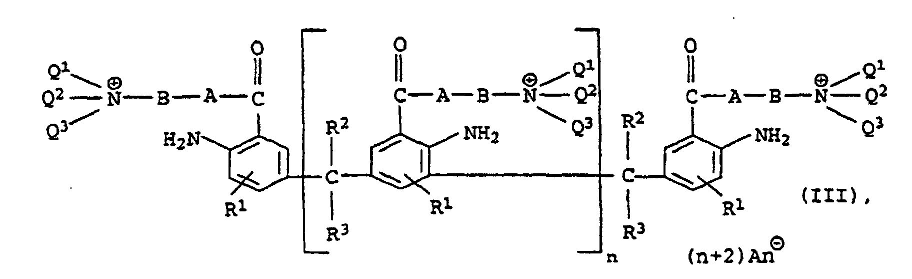 dans laquelle
n, R¹, R², R³, A, B, Q¹, Q², Q³ et An⊖ prennent la signification indiquée dans la revendication 1,
sur (n+2)/2 à n+2 équivalents molaires d'un composant de copulation de formule IIa' 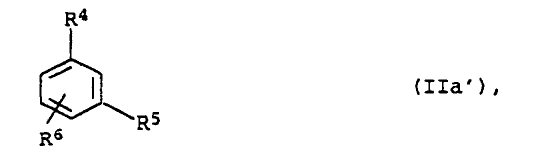
dans laquelle
R⁴ et R⁵ représentent, indépendamment l'un de l'autre, un groupe hydroxy, amino, morpholino ou mono-ou di-(alkyle en C₁ à C₆)-amino, dont les résidus alkyle peuvent éventuellement être substitués par un groupe hydroxy, amino, cyano, (alcoxy en C₁ à C₆)-carbonyle, carbamoyle ou mono- ou di-(alkyle C₁ à C₆)-carbamoyle et éventuellement interrompus par un groupe oxy, et
R⁶ représente un atome d'hydrogène, un groupe alcoxy en C₁ à C₆ (dont le reste alkyle est éventuellement interrompu par un groupe oxy), mono- ou di-(alkyle en C₁ à C₆)-amino, (alcoxy en C₁ à C₆)-carbonyle, carbamoyle, mono- ou di-(alkyle C₁ à C₆)-carbamoyle, sulfamoyle, mono-ou di-(alkyle C₁ à C₆)-sulfamoyle.

5. Colorants azoïques cationiques, pouvant être obtenus par copulation azoïque d'un équivalent molaire d'une diamine de formule III' (n=0) avec 1 à 2 équivalents molaires d'un composant de copulation de formule IIa'.

6. Procédé de préparation de colorants azoïques cationiques selon les revendications 4 et 5, dans lequel on couple 1 équivalent molaire d'amine sur (n+2)/2 à n+2 équivalents molaires d'un composant de copulation de formule IIa'.

7. Amines de formule générale III 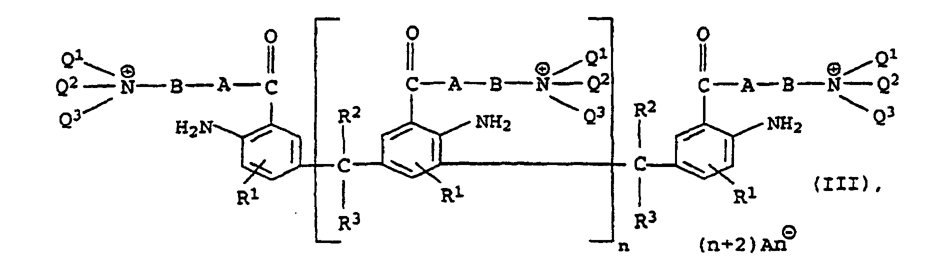 dans laquelle
n, R¹, R², R³, A, B, Q¹, Q², Q³ et An⊖ prennent la signification indiquée dans la revendication 1.

8. Mélanges de colorants, contenant plusieurs colorants de formule I selon les revendications 1 à 3, qui diffèrent par leur nombre de motifs récurrents.

9. Mélanges de colorants, contenant plusieurs colorants de formule I selon les revendications 1 à 3 ainsi qu'un ou plusieurs colorants de formule VI 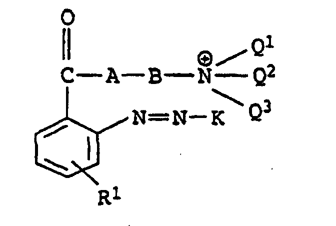 dans laquelle
R¹, K, A, B, Q¹, Q², Q³ et An⊖ prennent la signification indiquée dans la revendication 1.

10. Utilisation des colorants azoïques cationiques et de leurs mélanges selon les revendications 1 à 5 et 8 et 9 pour la teinture ou l'impression de matière polymère.
